# EUROPEAN PATENT SPECIFICATION

(11) **EP 3 968 628 B1**
(45) Date of publication and mention of the grant of the patent: **22.11.2023**
(21) Application number: 20830848.6
(22) Date of filing: 24.04.2020
(51) Int. Cl.: H04N 9/31, H04N 5/74, G03B 21/00, G03B 21/14, G09G 5/00

(54) **PROJECTION SYSTEM**
PROJEKTIONSSYSTEM
SYSTÈME DE PROJECTION

(30) Priority: 25.06.2019 JP 2019117375
(43) Date of publication of application: 16.03.2022
(73) Proprietor: Panasonic Intellectual Property Management Co., Ltd., Osaka-shi, Osaka 540-6207 (JP)
(72) Inventor: SAITO, Tomoyuki, Osaka-shi, Osaka 540-6207 (JP); KINIWA, Yuji, Osaka-shi, Osaka 540-6207 (JP); HIRANO, Tetsushi, Osaka-shi, Osaka 540-6207 (JP); SASAKI, Yuuhi, Osaka-shi, Osaka 540-6207 (JP)
(74) Representative: Eisenführ Speiser
(86) International application number: PCT/JP2020/017625
(87) International publication number: WO 2020/261741

(56) References cited:
- EP-B1- 2 177 163
- WO-A1-2019/103052
- JP-A- 2011 155 412
- JP-A- 2016 149 618
- JP-A- 2017 158 042
- US-A1- 2015 381 908
- US-A1- 2016 252 716
- DAI XIAOBIN ET AL: "A fast vein display device based on the camera-projector system", 2013 IEEE INTERNATIONAL CONFERENCE ON IMAGING SYSTEMS AND TECHNIQUES (IST), 1 October 2013 (2013-10-01), pages 146-149, XP055901473, DOI: 10.1109/IST.2013.6729680 ISBN: 978-1-4673-5790-6 Retrieved from the Internet: URL:https://ieeexplore.ieee.org/stampPDF/g etPDF.jsp?tp=&arnumber=6729680&ref=aHR0cHM 6Ly9pZWVleHBsb3JlLmllZWUub3JnL2RvY3VtZW50L zY3Mjk2ODA=>

## Description

### TECHNICAL FIELD

The present disclosure relates to a projection system that projects a projection image based on a captured image of a subject.

### BACKGROUND ART

An imaging system according to EP 2 1 77 163 B1 illuminates an object with infrared light to enhance visibility of buried structure beneath the surface of the object, and projects a visible light image of the buried structure onto the surface of the object. The system may include an infrared light source for generating the infrared light and a structure for diffusing the infrared light. The diffusing structure may include one or more layers of diffusing material for diffusing the light. The system further includes a video imaging device for receiving the infrared light reflected from the object and for generating a video image of the buried structure based on the reflected infrared light. The buried structure may be a subcutaneous blood vessel. A calibration procedure is described as well as embodiments for ensuring that the object is maintained in focus at the correct distance.

WO 2019/103052 A1 discloses a surgery support system in which a non-visible imaging unit captures a non-visible light image of a patient. The projection unit projects a projection image based on the non-visible light image on the patient using visible light.

An imaging system according to US 2015/0381908 A1 comprises a light field camera for recording a hyperspectral light field. The system also comprises a light projector for projecting a light field in visible light. The camera and the projector share a common optical axis. The projector projects a light field based on the hyperspectral light field captured by the light field camera.

PTL 1 discloses an optical imaging system used in the medical field. The optical imaging system of PTL 1 includes an electronic imaging device that captures an image of an operative field, a projector that projects a visible light image of a result of capturing an image of the operative field during a surgical operation, and an optical element that aligns optical axes of the electronic imaging device and the projector with the same optical axis. In PTL 1, before a surgical operation is performed, a test sample is previously placed, and a captured image of the test sample is captured, and in addition, by adjusting, while generating a projection image corresponding to the captured image, a correspondence relation between the captured image and the projection image on the same optical axis, calibration is performed to accurately project the projection image at the time of surgical operation.

### Citation List

### Patent Literature

PTL 1: U.S. Patent Publication No. 2008/0004533

### SUMMARY OF THE INVENTION

### Technical problem

An object of the present disclosure is to provide a projection system in which a projection image based on a captured image is projected and in which it is easier to adjust a positional relationship between the captured image and the projection image.

### Solution to problem

A projection system according to the present disclosure is defined in claim 1.

### Advantageous effect of invention

According to the projection system of the present disclosure, the positional relationship between the captured image and the projection image can be easily adjusted in the projection system that projects the projection image based on the captured image.

### BRIEF DESCRIPTION OF DRAWINGS

FIG. 1 is a schematic diagram illustrating a configuration of a surgical operation support system according to a first exemplary embodiment.
FIG. 2 is a block diagram illustrating a configuration of a head device in the surgical operation support system.
FIG. 3 is a functional block diagram illustrating an example of a normal mode of a projection controller in the surgical operation support system.
FIG. 4 is a flowchart for describing an operation in the normal mode in the surgical operation support system.
FIG. 5A is a diagram illustrating an example of a state of an operative field when an image is not yet projected in the surgical operation support system.
FIG. 5B is a diagram illustrating an example of a state of the operative field when an image is projected in the surgical operation support system.
FIG. 6 is a functional block diagram illustrating an example of a position adjustment mode of the projection controller in the surgical operation support system.
FIG. 7 is a flowchart illustrating an example of an operation in the position adjustment mode in the surgical operation support system.
FIG. 8 is a diagram illustrating an example of image data for projecting a marker image in the position adjustment mode.
FIG. 9A is a diagram illustrating an example of a captured image when the position adjustment mode is running but correction is not yet performed.
FIG. 9B is a diagram illustrating an example of a captured image when the position adjustment mode is running and after correction is performed.
FIG. 10 is a diagram illustrating a display example of a monitor in the position adjustment mode.
FIG. 11 is a flowchart illustrating an example of an automatic correction process in the position adjustment mode.
FIG. 12 is a diagram illustrating an example of image data for projecting a marker image for automatic correction.
FIG. 13A is a diagram illustrating a display example in an operation of a first position adjustment in the surgical operation support system.
FIG. 13B is a diagram illustrating a display example in an operation of a second position adjustment in the surgical operation support system.
FIG. 13C is a diagram illustrating a display example in an operation of a third position adjustment in the surgical operation support system.
FIG. 13D is a diagram illustrating a display example in an operation of a fourth position adjustment in the surgical operation support system.
FIG. 14 is a diagram illustrating an interpolation method of a projection image in the normal mode of the surgical operation support system.
FIG. 15 is a functional block diagram illustrating a first variation of the position adjustment mode in the surgical operation support system.
FIG. 16 is a diagram illustrating an example of image data for projecting a marker image according to a second variation.

### DESCRIPTION OF EMBODIMENTS

Hereinafter, exemplary embodiments will be described in detail with reference to the drawings as appropriate. However, unnecessarily detailed description is sometimes omitted. For example, the detailed description of already well-known matters and the redundant description of a configuration substantially identical to the already-described configuration is omitted in some cases. This is to avoid the following description from being unnecessarily redundant and thus to help those skilled in the art to easily understand the description.

The applicant provides the accompanying drawings and the following description such that those skilled in the art can fully understand the present disclosure, and therefore does not intend to limit the subject matters described in the claims by the accompanying drawings or the following description.

### (First exemplary embodiment)

A surgical operation support system will be described as a specific example of the projection system according to the present disclosure.

### 1. Configuration

### 1-1. Outline of surgical operation support system

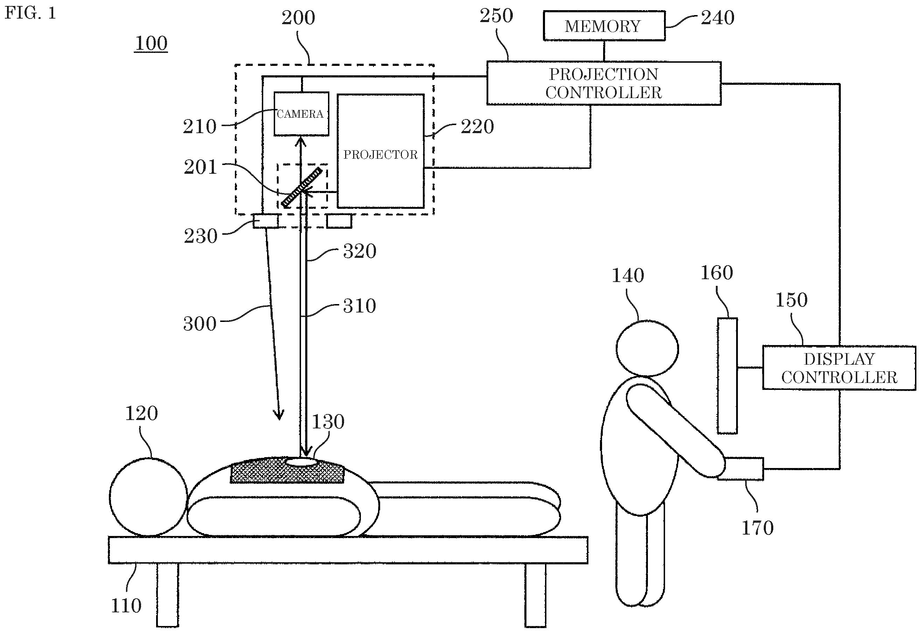

An outline of a surgical operation support system according to a first exemplary embodiment will be described with reference to FIG. 1. FIG. 1 is a schematic diagram illustrating a configuration of surgical operation support system 100 according to the first exemplary embodiment.

Surgical operation support system 100 includes camera 210, projector 220, and excitation light source 230. Surgical operation support system 100 is a system that visually supports, by using a projection image, a surgical operation performed on a patient by a medical doctor and the like in an operating room or the like. In the case of using surgical operation support system 100, a photosensitive substance is previously administered to patient 120 to undergo a surgical operation.

The photosensitive substance is a substance that emits fluorescent light by reaction with excitation light. As the photosensitive substance, for example, indocyanine green (ICG) or the like is used. In the present exemplary embodiment, a case will be described where ICG is used as an example of the photosensitive substance. Being irradiated with excitation light in an infrared region in the vicinity of wavelengths of 760 nm to 780 nm, ICG emits fluorescent light in an infrared region of wavelengths of 800 nm to 860 nm.

When administered to patient 120, the photosensitive substance accumulates in affected part 130 where blood flow or lymphatic flow is sluggish. Therefore, it is possible to identify an area of affected part 130 by detecting an area emitting fluorescent light by reaction with applied excitation light 300.

However, since the fluorescent light emitted from affected part 130 is weak and a wavelength band of the fluorescent light is in an invisible region or in the vicinity of the invisible region, it is difficult for the medical doctor and the like to identify the area of affected part 130 even when the doctor or the like visually observes the operative field. Therefore, surgical operation support system 100 identifies, by using camera 210, the area of affected part 130 that emits fluorescent light 310. Further, projector 220 irradiates affected part 130 with projection light 320 of visible light such that a human can visually recognize affected part 130. As a result, the projection image is projected to visualize the identified area of affected part 130, so that the medical doctor or the like performing the surgical operation can be supported to identify the area of affected part 130.

### 1-2. Configuration of surgical operation support system

A configuration of surgical operation support system 100 will be described below with reference to FIG. 1. Surgical operation support system 100 is used being placed in an operating room of a hospital. Surgical operation support system 100 includes head device 200, memory 240, and projection controller 250.

Although not illustrated in the drawings, surgical operation support system 100 includes a mechanism for changing a position of head device 200. For example, surgical operation support system 100 includes a drive arm mechanically connected to head device 200 and casters of a base on which a set of surgical operation support system 100 is placed. With the above mechanism, head device 200 is disposed vertically above operating table 110 on which patient 120 is placed, or is disposed above the operating table at a certain angle from the vertical direction. In addition, operating table 110 may include a drive mechanism capable of changing a height and orientation of operating table 110.

Head device 200 is an example of a projection device in which camera 210, projector 220, and excitation light source 230 are integrally assembled together with an optical system such as dichroic mirror 201. Details of the configuration of head device 200 will be described later.

Memory 240 is a storage medium that projection controller 250 appropriately access when performing various calculations. Memory 240 includes, for example, a random access memory (RAM) and a read only memory (ROM). Memory 240 is an example of a storage unit.

Projection controller 250 integrally controls each unit constituting surgical operation support system 100. Projection controller 250 is an example of a controller. Projection controller 250 is electrically connected to camera 210, projector 220, excitation light source 230, and memory 240, and outputs control signals for controlling each unit. Projection controller 250 includes, for example, a central processing unit (CPU), and achieves functions of projection controller 250 by executing a predetermined program. Note that the function of projection controller 250 may be achieved by a dedicated electronic circuit or a reconfigurable electronic circuit, that is, a field programmable gate array (FPGA), an application specific integrated circuit (ASIC), or the like.

In the present exemplary embodiment, surgical operation support system 100 includes display controller 150, monitor 160, and operation unit 170.

Display controller 150 includes, for example, a personal computer (PC), and is connected to projection controller 250. Display controller 150 includes, for example, a CPU, and performs image processing or the like for controlling an image to be displayed on monitor 160. A controller of this system 100 may include display controller 150 and projection controller 250. Display controller 150 further includes an internal memory (ROM, RAM, or the like), which is an example of a storage unit.

Monitor 160 includes a display surface that is configured with, for example, a liquid crystal display or an organic electroluminescence (EL) display and displays an image. Monitor 160 is an example of a display unit.

Operation unit 170 is an input interface that receives various user operations that are input from user 140. Operation unit 170 includes, for example, various operation members such as direction instruction keys, a button, a switch, a keyboard, a mouse, a touch pad, and a touch panel.

User 140 can check a captured image captured by camera 210 on monitor 160, for example, during a surgical operation. Further, user 140 can adjust various settings of the projection image.

### 1-3. Configuration of head device

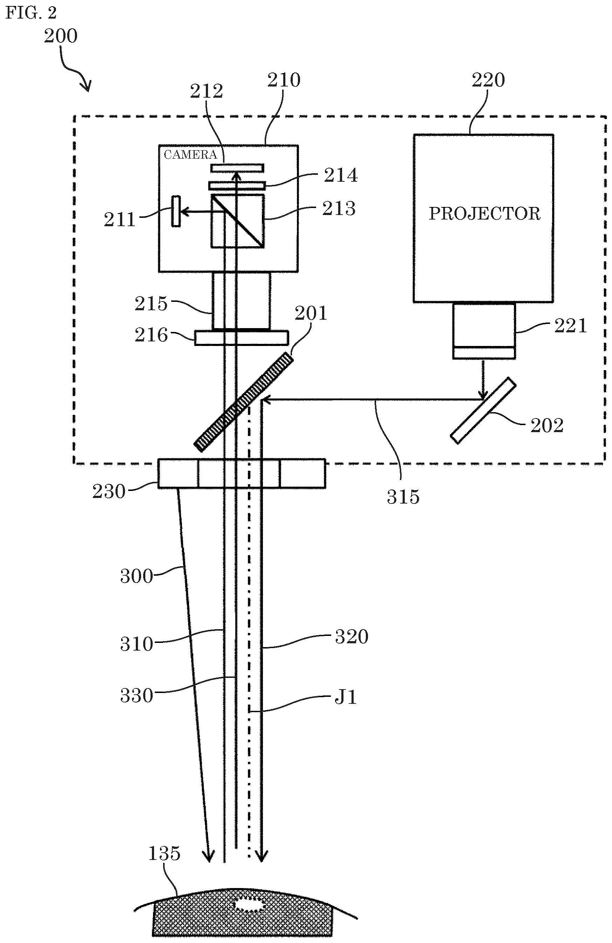

Details of a configuration of head device 200 will be described with reference to FIG. 2. FIG. 2 is a block diagram illustrating the configuration of head device 200 in surgical operation support system 100. Head device 200 includes excitation light source 230, camera 210, zoom lens 215, optical filter 216, projector 220, projection lens 221, dichroic mirror 201, and mirror 202. Head device 200 is disposed at a position at a distance (height) of, for example, 1 m to a subject such as operative field 135 and the like.

Excitation light source 230 is a light source device that emits excitation light 300 to cause the photosensitive substance to emit fluorescent light. In the present exemplary embodiment, since ICG is used as the photosensitive substance, excitation light source 230 emits excitation light 300 having a wavelength band (for example, about 760 nm to 780 nm) including an excitation wavelength of ICG. Excitation light source 230 is an example of an illuminator. Excitation light source 230 switches between on and off of radiation of excitation light 300 according to the control signal from projection controller 250. Note that excitation light source 230 may be configured separately from head device 200.

Camera 210 captures an image of a subject including operative field 135 of patient 120 and the like to generate a captured image. Camera 210 transmits image data representing the generated captured image to projection controller 250. In the present exemplary embodiment, as illustrated in FIG. 2, camera 210 includes imaging sensor 211 for infrared light, imaging sensor 212 for visible light, prism 213, and optical filter 214. Each imaging sensor 211, 212 includes, for example, a complementary metal-oxide semiconductor (CMOS) image sensor or a charge-coupled device (CCD) image sensor.

For example, prism 213 has such optical characteristics that a light component in an infrared region is reflected and a light component in the visible region (or outside an infrared region) is transmitted. On the reflective surface side of prism 213, there is disposed imaging sensor 211 for infrared light. Prism 213 is disposed between imaging sensor 212 for visible light and zoom lens 215.

Optical filter 214 includes, for example, a band pass filter or a low pass filter, transmits light in the visible region, and blocks light outside the visible region (for example, ultraviolet region). Optical filter 214 is disposed between imaging sensor 212 for visible light and prism 213.

Imaging sensor 211 for infrared light captures an image of infrared light (an example of invisible light) including a wavelength band of 800 nm to 860 nm that is fluorescent light of ICG, and generates an invisible light image as a captured image. Imaging sensor 211 for infrared light may include a filter or the like that blocks light other than infrared light. Imaging sensor 211 for infrared light is an example of an invisible image capturing unit.

Imaging sensor 212 for visible light performs imaging by visible light including a part of the visible region or the entire visible region, and generates, for example, a monochrome visible light image as a captured image. Imaging sensor 212 for visible light is an example of a visible image capturing unit. The visible image capturing unit is not limited to capturing a monochrome image, and may be configured to be able to capture a captured image in RGB, for example. For example, the visible image capturing unit may include one CMOS sensor or the like in which RGB color filters are provided for respective ones of pixels, or may include three CMOS sensors or the like each for capturing an image in one of RGB colors.

Prism 213 and optical filter 214 are an example of an internal optical system provided inside camera 210. The internal optical system of camera 210 is not limited to the above example. For example, a member for adjusting an optical path length may be disposed between each imaging sensor 211, 212 and prism 213.

Further, instead of the above-described optical characteristics, prism 213 may have such optical characteristics that invisible light such as infrared light is reflected and visible light is mainly transmitted, or may have such optical characteristics that invisible light and visible light are both reflected. The arrangement of imaging sensors 211, 212 are appropriately changed depending on the optical characteristics of prism 213.

Zoom lens 215 is attached to camera 210 and condenses light from outside inside camera 210. Zoom lens 215 adjusts an angle of view (zoom value), a depth of field, focusing, and the like of camera 210. Zoom lens 215 includes various lens elements and a diaphragm.

The zoom value of zoom lens 215, an f-number of the diaphragm, and the like can be set from outside, for example. Zoom lens 215 is an example of an imaging optical system. The imaging optical system is not limited to zoom lens 215, and may include, for example, an internal optical system of camera 210 or various external optical elements, or may be incorporated in camera 210 as an internal optical system.

For example, as illustrated in FIG. 2, optical filter 216 is disposed on an incident plane of zoom lens 215. Optical filter 216 includes a band-cut filter that blocks a wavelength band component of 680 nm to 825 nm including wavelengths of 760 nm to 780 nm of the excitation light in the incident light.

Projector 220 is a projector using a dynamic light processing (DLP) system, a 3 liquid crystal display (3LCD) system, a liquid crystal on silicon (LCOS) system, or the like. Projector 220 emits projection light 315 to project, with visible light, a projection image based on a video signal being input from projection controller 250. Projector 220 is an example of the projection unit. Projector 220 includes, for example, a light source, an image forming unit, an internal optical system, and the like.

Light source of projector 220 includes, for example, a laser diode (LD) or a light emitting diode (LED). An image forming unit of projector 220 includes a spatial light modulation element such as a digital micromirror device (DMD) or a liquid crystal display (LCD), and forms an image based on a video signal from projection controller 250, on an image forming plane of the spatial light modulation element. Projector 220 spatially modulates light from the light source in accordance with the formed image to generate projection light 315, and emits projection light 315 through the internal optical system.

Projector 220 may have a projection control circuit that achieves functions specific to projector 220, such as a trapezoidal correction function and a lens shift function. Further, the above-described functions may be achieved in projection controller 250. Further, projector 220 may be a laser scanning type, and may be configured to include a micro electro mechanical system (MEMS) mirror or a galvano mirror that can be driven in a scanning direction.

Projection lens 221 is disposed to be optically coupled to the internal optical system of projector 220. Projection lens 221 is configured as, for example, a tele conversion lens, and extends a focal length of projector 220 to a telephoto side.

Mirror 202 is disposed between projection lens 221 and dichroic mirror 201. In addition to or instead of mirror 202 and projection lens 221, various optical elements may be disposed in an optical path from projector 220 to dichroic mirror 201.

Dichroic mirror 201 is an example of a light guide having optical characteristics of selectively transmitting or reflecting incident light, depending on a wavelength band of the light. For example, a transmittance and a reflectance for infrared light of dichroic mirror 201 are respectively set to 100% and 0% within a tolerance range. Further, in the present exemplary embodiment, the reflectance and the transmittance for visible light are set such that dichroic mirror 201 transmits visible light with a transmittance in a range smaller than the reflectance of visible light. The transmittance of visible light of dichroic mirror 201 is preferably 5% or less. For example, the reflectance of visible light of dichroic mirror 201 is 99%, and the transmittance of visible light is 1%.

That is, dichroic mirror 201 limits visible light with which imaging sensor 212 for visible light can capture an image to 5% or less. The value 5% or less may be achieved not only by dichroic mirror 201 but also may be achieved by combining members on the optical path of imaging. For example, the value 5% or less may be achieved, as a whole, not only by dichroic mirror 201 but also by a filter additionally provided on optical filter 216, prism 213, or the like.

As illustrated in FIG. 2, due to the above optical characteristics, dichroic mirror 201 transmits fluorescent light 310 and the like directed to camera 210 via zoom lens 215 and the like, and reflects most (more than half) of projection light 315 radiated from projector 220. Reflected projection light 320 is applied onto operative field 135. In the present exemplary embodiment, dichroic mirror 201 guides light such that the following optical axes coincide with each other on optical axis J1: an optical axis of incident light such as fluorescent light 310 from operative field 135 entering camera 210; and an optical axis of projection light 320 for projecting the projection image onto operative field 135. This arrangement can reduce positional deviation of the projection image based on the captured image by camera 210.

Note that a tolerance may be appropriately set to the coincidence of optical axes in the present disclosure. For example, the optical axes may coincide with each other with such a tolerance that an angular error is within a range of ±5 degrees or an interval between the optical axes is within a range of 1 cm. In addition, the optical characteristics of dichroic mirror 201 can be appropriately set depending on fluorescence properties and the like of the photosensitive substance to be used.

### 1-4. Functional configuration of projection controller

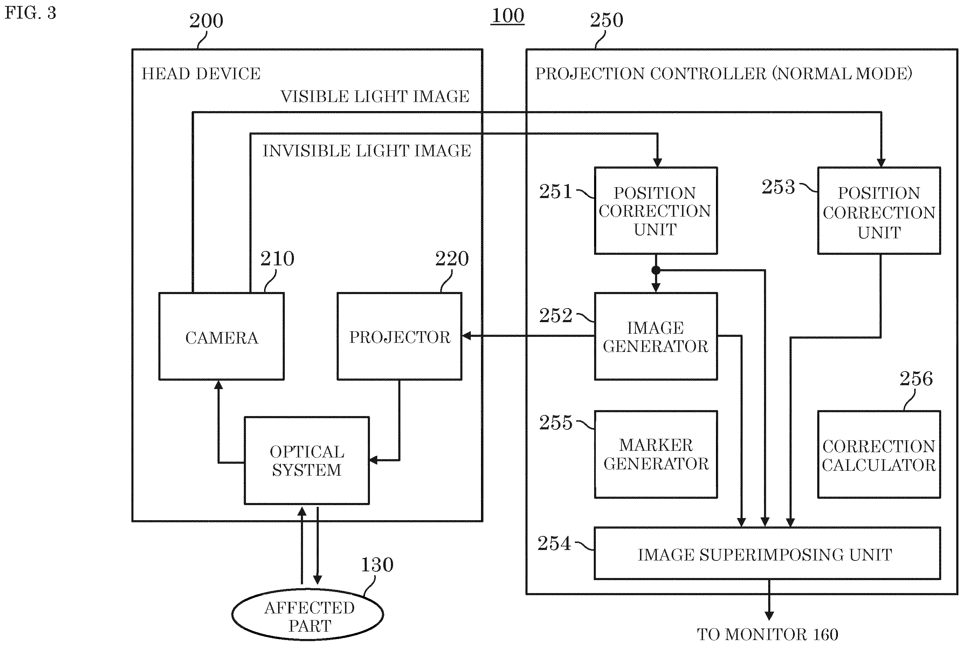

FIG. 3 is a functional block diagram illustrating an example of a normal mode of projection controller 250. Surgical operation support system 100 and projection controller 250 have the normal mode, which is an example of a first operation mode and a position adjustment mode, which is an example of a second operation mode.

Projection controller 250 includes, as functional components, position correction unit 251 for an invisible light image, image generator 252, position correction unit 253 for a visible light image, image superimposing unit 254, marker generator 255, and correction calculator 256.

Each of position correction units 251, 253 performs, on the captured image by camera 210, processing of correcting the position of a whole image in accordance with correction information set in advance. The correction information is an example of information representing a positional relationship between the captured image and the projection image, and includes various parameters defining various coordinate transformations.

Image generator 252 performs various types of image processing on the captured image corrected by position correction unit 251 for an invisible light image to generate image data (video signal) representing the projection image. The various types of image processing include binarization or multi-valuing, color conversion, and the like.

Image superimposing unit 254 performs image composition to superimpose the captured images, the projection image, and the like, and outputs the composed image to display controller 150 or monitor 160.

Marker generator 255 and correction calculator 256 operate in the position adjustment mode. Various operation modes and functions of projection controller 250 will be described later.

### 2. Operation

An operation of surgical operation support system 100 according to the present exemplary embodiment will be described below.

### 2-1. Operation in normal mode

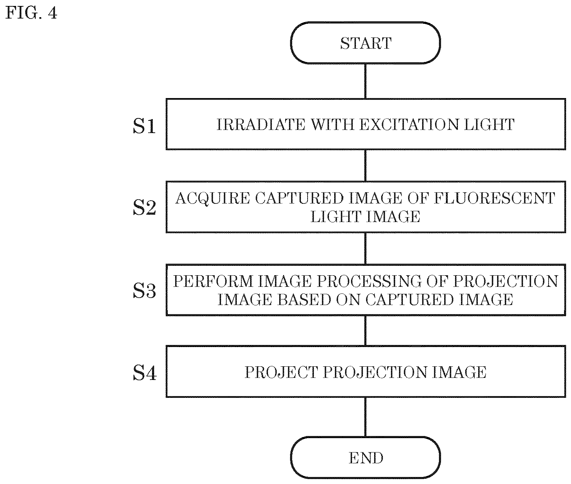

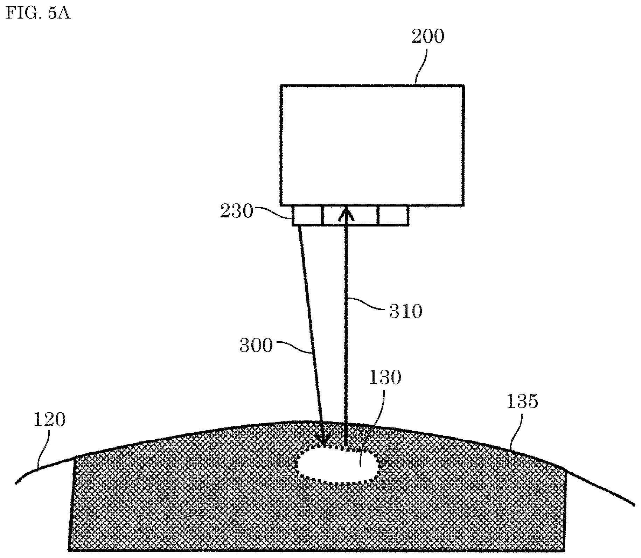

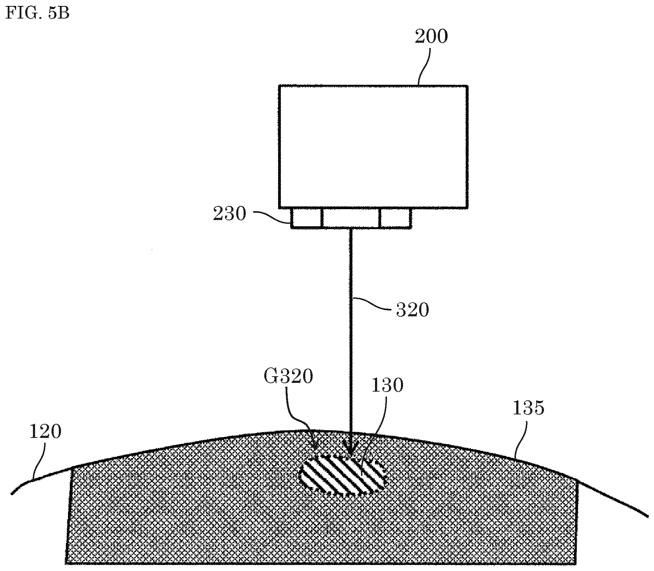

An operation in the normal mode of surgical operation support system 100 will be described with reference to FIGS. 4, 5A, and 5B. The normal mode is an operation mode for performing a basic projection operation for supporting a surgical operation in surgical operation support system 100.

FIG. 4 is a flowchart for describing the operation in the normal mode in surgical operation support system 100. FIG. 5A illustrates a state of operative field 135 in surgical operation support system 100 before a projection operation in the normal mode is performed. FIG. 5B illustrates a state where the projection operation is performed on operative field 135 of FIG. 5A. Each processing illustrated in the flowchart of FIG. 4 is performed by projection controller 250.

In the flowchart of FIG. 4, projection controller 250 drives excitation light source 230 to irradiate operative field 135 with excitation light 300 as illustrated in FIG. 5A (step S1). By the irradiation of excitation light 300, affected part 130 in operative field 135 emits fluorescent light, and fluorescent light 310 from affected part 130 enters head device 200.

In head device 200, as shown in FIG. 2, fluorescent light 310 passes through dichroic mirror 201, and passes through optical filter 216 of camera 210. As a result, camera 210 receives fluorescent light 310 with imaging sensor 211 for infrared light. At this time, the reflected light of excitation light 300 is blocked by optical filter 216.

Next, projection controller 250 controls and causes, for example, camera 210 to capture an image of operative field 135, and acquires a captured image from camera 210 (step S2). The captured image acquired in step S2 includes the fluorescent light image generated by receiving fluorescent light 310 emitted from affected part 130.

Next, projection controller 250 functions as position correction unit 251 and image generator 252 to perform image processing for generating a projection image based on the acquired captured image (step S3). Projection controller 250 generates an image corresponding to the fluorescent light image in the captured image and outputs the generated image as a video signal to projector 220.

In the processing in step S3, referring to the correction information previously set and stored in memory 240, projection controller 250 first serves as position correction unit 251 and performs coordinate transformation such as shift, rotation, and enlargement and reduction on the acquired captured image. As a result, the position of the image is corrected. Projection controller 250 may further correct image distortion and the like.

Next, projection controller 250 performs, as image generator 252, binarization on a distribution of intensity of the received light in the corrected captured image on the basis of a predetermined threshold, and identifies an area considered to be an area of the fluorescent light image in the captured image. For example, image generator 252 generates an image representing a specific area corresponding to the fluorescent light image in the captured image by setting different colors to an inside and an outside of the identified area (step S3). For example, the inside of the identified area is set to a chromatic color such as blue, and the outside of the area is set to an achromatic color such as white.

Next, projection controller 250 controls projector 220 to project the projection image, based on the generated video signal (step S4). Under the control of projection controller 250, projector 220 generates projection light 315 representing the projection image in accordance with the video signal from projection controller 250, and emits projection light 315 to dichroic mirror 201 via projection lens 221 (see FIG. 2).

As illustrated in FIG. 2, dichroic mirror 201 reflects (most of) projection light 315, which is visible light, and emits projection light 320 along optical axis J1. As a result, as illustrated in FIG. 5B, head device 200 irradiates operative field 135 with projection light 320, and projection image G320 is projected onto affected part 130 in operative field 135. Projection image G320 is, for example, a single-color image.

The above process is repeatedly executed at a predetermined cycle (for example, 1/60 to 1/30 seconds).

Through the above process, projection controller 250 identifies the area of affected part 130 emitting fluorescent light on the basis of the captured image by camera 210, and projection image G320 of the visible light is projected from projector 220 onto affected part 130. As a result, in surgical operation support system 100, affected part 130 that is difficult to visually recognized can be visualized. Surgical operation support system 100 allows the medical doctor and the like to visually recognize the state of affected part 130 in real time.

In the above example, projection image G320 is in a single color. For example, projection controller 250 may generate a multi-gradation projection image by determining the area of the fluorescent light image in the captured image, at multiple levels using a plurality of thresholds. Further, projection controller 250 may generate the projection image such that the distribution of the received light intensity in the captured image is reproduced continuously. The projection image may be generated in multiple colors or in full color.

### 2-1-1. Visible image capturing function

In surgical operation support system 100 according to the present exemplary embodiment, in addition to the fluorescent light image (step S2 in FIG. 4) for generating the projection image being captured as described above, an image of the operative field and the like is captured in visible light. A visible image capturing function in surgical operation support system 100 will be described with reference to FIG. 2.

Visible light 330 entering head device 200 of surgical operation support system 100 includes a portion of external light reflected by a subject such as the operative field, a reflected light portion of projection light 320, and other light. Visible light 330 enters dichroic mirror 201 in head device 200.

Dichroic mirror 201 of the present exemplary embodiment transmits a part of incident visible light 330 and allows the part to enter zoom lens 215 through optical filter 216. Optical filter 216 according to the present exemplary embodiment transmits incident visible light 330 with a predetermined transmittance. Zoom lens 215 adjusts a light flux of incident visible light 330 in accordance with the set zoom value and diaphragm value, and allows the light flux to enter camera 210.

In camera 210, prism 213 transmits incident visible light 330. Imaging sensor 212 for visible light receives visible light 330 having passed through prism 213. As a result, imaging sensor 212 for visible light captures an image of visible light 330 from the subject and the like. Camera 210 outputs the visible light image that is a result of the image capturing by imaging sensor 212 for visible light to, for example, at least one of display controller 150 and projection controller 250 (see FIG. 1).

Further, when infrared light such as fluorescent light 310 enters (see FIG. 2) prism 213, prism 213 reflects the incident infrared light and guides the reflected infrared light to imaging sensor 211 for infrared light. Camera 210 can simultaneously capture an invisible light image on imaging sensor 211 for infrared light and a visible light image on imaging sensor 212 for visible light.

The above visible image capturing function is used, for example, to display or record the state of the operative field during a surgical operation. For example, display controller 150 (FIG. 1) displays the visible light image on monitor 160, and memory 240 or the like records the visible light image. In addition, various display modes can be set in surgical operation support system 100 by performing image processing of superimposing the invisible light image or performing other processing on the visible light image. Further, the visible image capturing function can also be used to correct positional deviation of projection image G320 (FIG. 5B).

### 2-2. Position adjustment mode

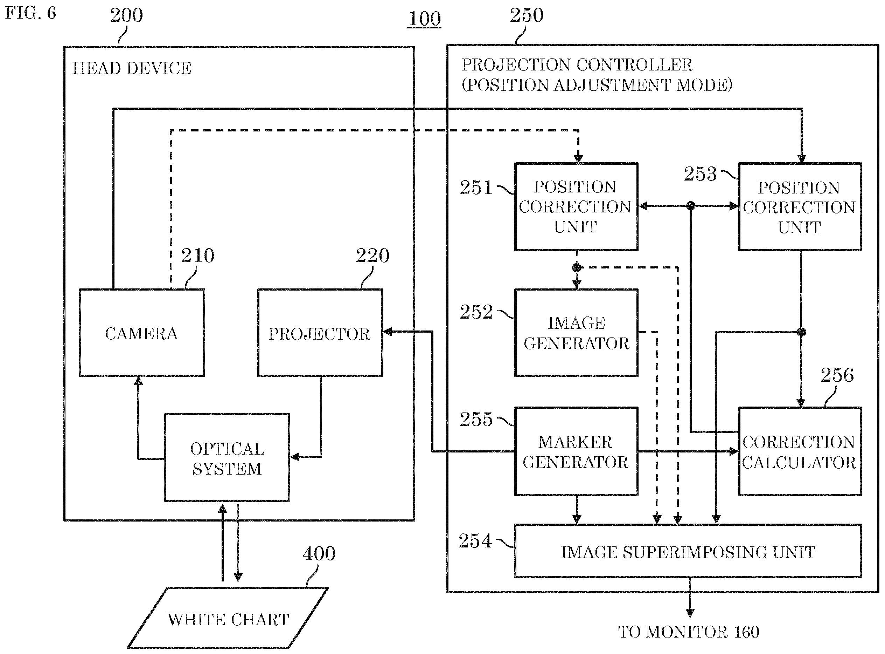

An outline of the operation of the position adjustment mode in present system 100 will be described with reference to FIG. 6. FIG. 6 is a functional block diagram illustrating an example of the position adjustment mode of projection controller 250 in present system 100.

As described above, in the normal mode, surgical operation support system 100 projects projection image G320 from projector 220, based on the captured image by camera 210, thereby visualizing affected part 130 that is difficult to be visually recognized during a surgical operation (See FIGS. 5A and 5B). In this system 100, the position adjustment mode is provided in which positioning between the captured image by camera 210 and the projection image by projector 220 is performed in advance such that affected part 130 can be visualized without error by way of projection image G320 at the time of operation of the normal mode, for example, during a surgical operation. The positioning in the position adjustment mode is successively performed, for example, every time a surgical operation is scheduled to be performed.

One typical method for positioning is performed as follows: various samples for test are prepared instead of, for example, affected part 130; a projection image is once generated based on a captured image of the sample and is projected on the sample; and a feedback is performed so as to minimize a deviation of the projection image with respect to the sample. However, by such a method, a situation arises in which the deviation of the projection image regenerated after the feedback with respect to the sample hardly converges; therefore, the positional relationship between the captured image and the projection image cannot be easily adjusted.

To address this issue, in the position adjustment mode of the present exemplary embodiment, an order of operations of camera 210 and projector 220 is reversed such that roles of camera 210 and projector 220 are changed from the roles in the above-described normal mode. Specifically, as illustrated in FIG. 6, projector 220 first projects a predetermined marker image generated by marker generator 255 onto white chart 400 or the like configured with a white plate, for example. Next, camera 210 captures an image of the projected marker image, and correction calculator 256 performs calculation related to a positional deviation of the marker image in the captured image by using the marker image in a projection source as a reference.

By this operation of the position adjustment mode, it is possible to easily adjust the positional relationship between the projection image and the captured image by using the marker image in the projection source (or the original image data for projection) as a reference. Hereinafter, details of the operation of the position adjustment mode in this system 100 will be described.

### 2-2-1. Operation of position adjustment mode

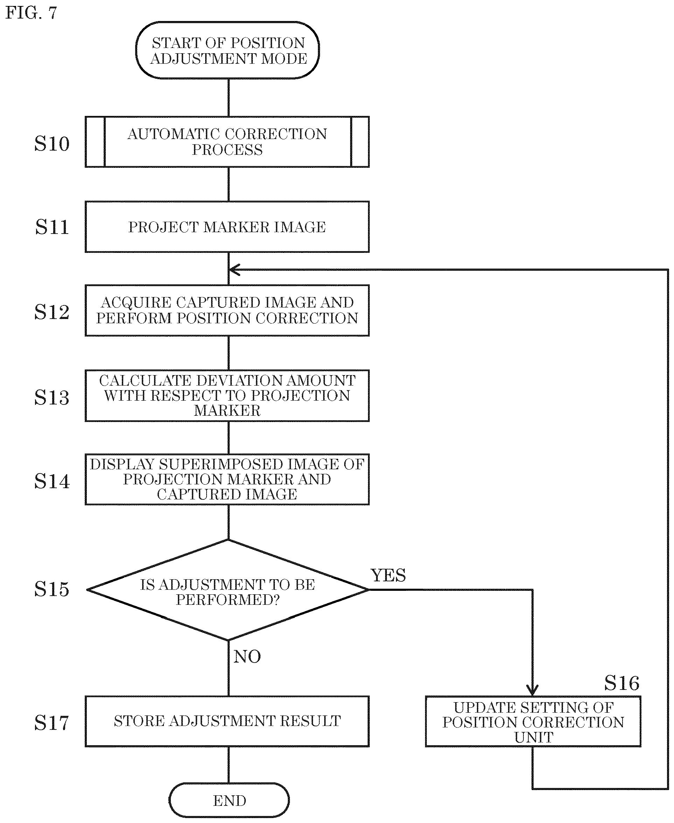

An operation of the position adjustment mode in the present exemplary embodiment will be described with reference to FIGS. 7 to 10.

FIG. 7 is a flowchart illustrating an example of the operation in the position adjustment mode in surgical operation support system 100. This flowchart starts, when a user operation to start the position adjustment mode is input, for example, on operation unit 170. Each processing illustrated in this flowchart is executed by projection controller 250, for example.

Projection controller 250 first performs an automatic correction process (step S10). The automatic correction process performs the following steps: automatically adjusting the positional relationship between the projection image and the captured image by using the marker image; and initializing position correction unit 253 and position correction unit 251 such that the positional deviation of the captured image falls within a predetermined range of tolerance. Details of the automatic correction process will be described later in detail.

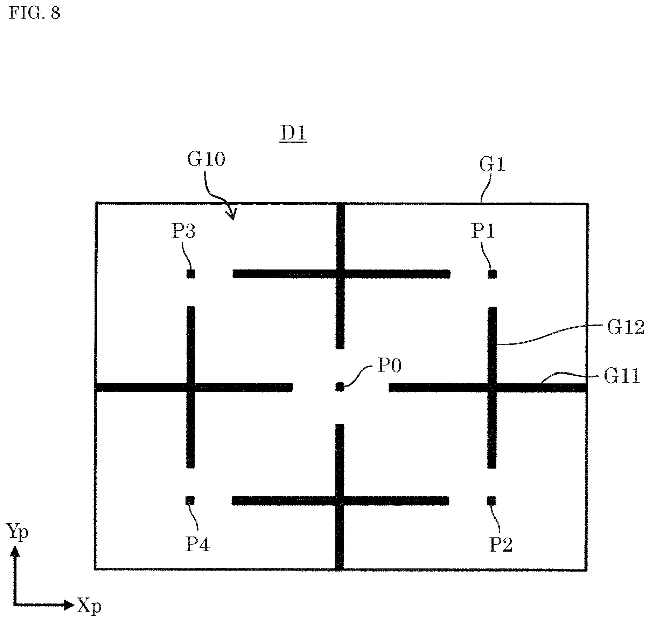

Next, projection controller 250 functions as marker generator 255 and controls and causes projector 220 to project a marker image (step S11). In step S11, projection controller 250 reads from memory 240 image data for projection corresponding to the marker image, and outputs the read-out image data to projector 220. FIG. 8 illustrates an example of marker image G1 represented by image data D1 for projection in step S11.

As illustrated in FIG. 8, the image data for projection such as image data D1 has a projection coordinate (Xp, Yp) that is a two-dimensional coordinate defining a position in each projection image. Marker image G1 defined by image data D1 includes projection marker G10 and a region other than projection marker G10. Projection marker G10 is an example of a marker that is set at a reference position on the projection coordinate (Xp, Yp).

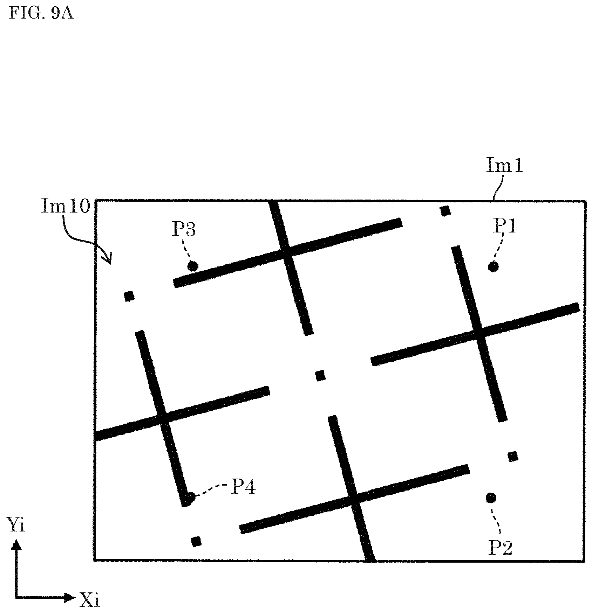

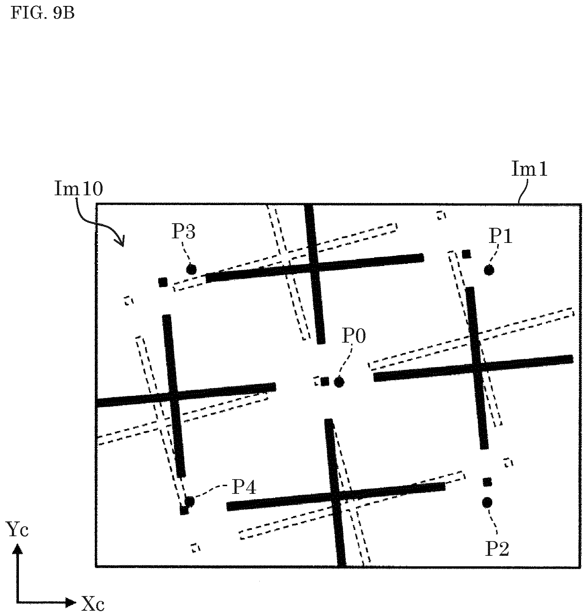

Next, projection controller 250 acquires from camera 210 a captured image in which projected marker image G1 is captured, and performs as position correction unit 253 processing on the captured image (step S12). In step S12, projection controller 250 acquires, by a process as position correction unit 253, the captured image after being corrected by, for example, the automatic correction process (step S10). FIGS. 9A and 9B each illustrate an example of captured image Im1 before and after the correction in step S12.

FIG. 9A illustrates an example of captured image Im1 captured by camera 210 when marker image G1 of FIG. 8 is projected. FIG. 9B illustrates an example of a state in which position correction unit 253 that is initially set in step S10 has processed captured image Im1 of FIG. 9A. Hereinafter, projection marker G10 appearing in captured image Im1 is referred to as a "captured marker".

In step S12, the processing of position correction unit 253 is performed such that captured image Im1 is coordinate-transformed from an imaging coordinate (Xi, Yi) at the time of image capturing by camera 210 to a corrected imaging coordinate (Xc, Yc) in accordance with the information indicating a previously set positional relationship. In the examples of FIGS. 9A and 9B, the position of captured marker Im10 on the corrected imaging coordinate (Xc, Yc) deviates from the position of projection marker G10 in FIG. 8 by an amount of a correction remainder of the automatic correction process (step S10) for the imaging coordinate (Xi, Yi).

Again with reference to FIG. 7, projection controller 250 refers, as correction calculator 256, to image data D1 for projection in memory 240, and calculates a deviation amount representing the positional deviation between captured marker Im10 on the imaging coordinate (Xc, Yc) and projection marker G10 on the projection coordinate (Xp, Yp) (step S13). For example, projection controller 250 performs image analysis on captured image Im1, and detects, on the imaging coordinate (Xc, Yc), a position of a specific portion of captured marker Im10 corresponding to a reference position of projection marker G10, in other words, detects a marker position. Projection controller 250 calculates an X-component deviation amount ΔX = (Xc - Xp) and a Y-component deviation amount ΔY = (Yc - Yp) for each of the detected one or more marker positions.

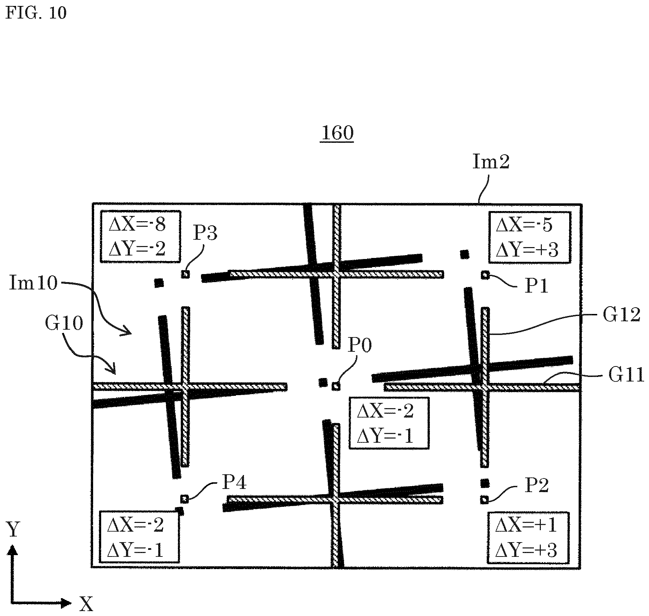

Further, projection controller 250 refers, as image superimposing unit 254, to image data D1 for projection and generates superimposed image Im2 in which projection marker G10 is superimposed on captured image Im1, and projection controller 250 causes monitor 160 to display superimposed image Im2 via communication with display controller 150, for example (step S14). A display example of monitor 160 in step S14 is illustrated in FIG. 10.

The display example of FIG. 10 illustrates an example of superimposed image Im2 depending on captured image Im1 of FIG. 9B. For example, image superimposing unit 254 superimposes projection marker G10 on captured image Im1 such that captured image Im1 and projection marker G10 are respectively placed at (X, Y) = (Xc, Yc) and (X, Y) = (Xp, Yp) on a coordinate (X, Y) for display on monitor 160 Further, for example, as illustrated in the present display example, projection controller 250 makes the calculated deviation amounts (ΔX, ΔY) be displayed together with superimposed image Im2 in step S14.

The display as illustrated in FIG. 10 is performed to cause user 140 to check whether to perform further position adjustment for correction. User 140 can appropriately input, from operation unit 170, a user operation for performing position adjustment. Projection controller 250 determines whether the user operation input from operation unit 170 is an operation for position adjustment (step S15).

When the operation for position adjustment is input (step S15: YES), projection controller 250 updates the information set in position correction unit 253 and position correction unit 251 according to the user operation having been input (step S16), and projection controller 250 performs the processes in and after step S12 again. At this time, the imaging coordinate (Xc, Yc) of captured image Im1 is further corrected (step S12), and the display of superimposed image Im2 and the like are updated (step S14). For example, when a desired correction is achieved by repeating the operation for position adjustment, user 140 inputs to operation unit 170 an operation for completion of the position adjustment mode.

When the operation for completion of the position adjustment mode is input, projection controller 250 determines that the user operation having been input is not the operation for position adjustment (step S15: NO), and stores adjustment results (step S17). At this time, projection controller 250 records in memory 240 various types of information regarding position correction unit 253 for a visible light image and position correction unit 251 for an invisible light image. After that, the process according to the present flowchart ends.

By the above processes, position adjustment is performed by using projection marker G10 as a reference in such a manner that the imaging coordinate (Xc, Yc) is repeatedly corrected while constant marker image G1 is continuously projected from projector 220. For example, user 140 can easily reach a desired correction state by performing an operation for position adjustment so as to bring captured marker Im10 closer to projection marker G10 on superimposed image Im2 of monitor 160.

Further, by the above process, in steps S10 to S16, the settings are successively updated similarly between position correction unit 253 for a visible light image and position correction unit 251 for an invisible light image, and the adjustment results for both position correction units 251, 253 can be made similarly (step S17). Instead of the above method, projection controller 250 may update the settings of one position correction unit 253 in steps S10 to S16, and performs in step S17 setting for another position correction unit 251 such that both position correction units 251, 253 finally coincide with each other.

### 2-2-2. Automatic correction process

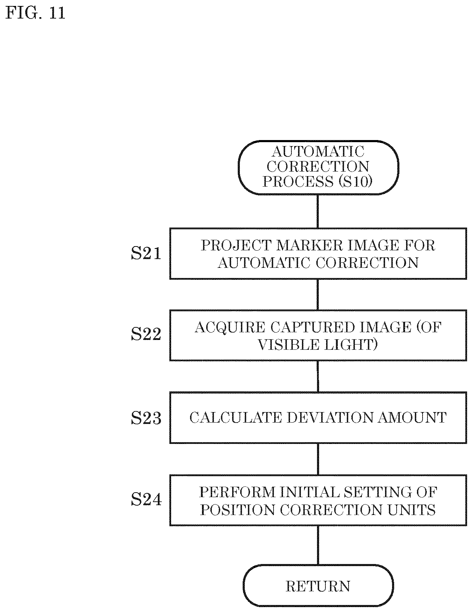

The automatic correction process (step S10) in the above position adjustment mode will be described in detail with reference to FIGS. 11 to 12. FIG. 11 is a flowchart illustrating an example of the automatic correction process in the position adjustment mode.

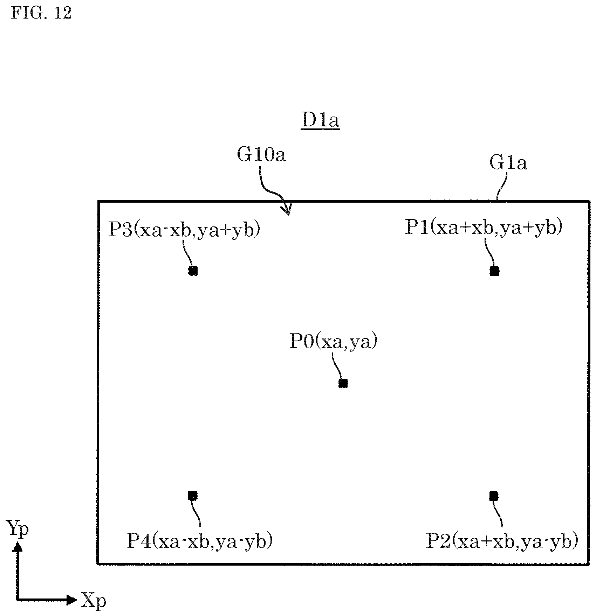

In the automatic correction process (step S10), projection controller 250 performs the same process as the above-described steps S11 to S13, while using, for example, a marker image for automatic correction instead of marker image G1 in FIG. 8 (steps S21 to S23). FIG. 12 illustrates an example of image data D1a of marker image G1a for automatic correction.

Marker image G1a illustrated as an example in FIG. 12 includes, as projection marker G10a, marker point P0 at a center position (Xp, Yp) = (xa, ya) on the projection coordinate (Xp, Yp) and marker points P1 to P4 at four marker positions (Xp, Yp) = (xa ± xb, ya ± yb). A size of each of marker points P1 to P4 is appropriately set to have one or more pixels. The positions of marker points P1 to P4 are examples of reference positions on the projection coordinate (Xp, Yp).

Further, in marker image G1a, projection marker G10a of the present example is set to have luminance lower than luminance of a region other than projection marker G10a. For example, in marker image G1a, the entire region other than projection marker G10a is set to white (that is, the highest luminance), and projection marker G10a is set to black (that is, the lowest luminance). With this arrangement, when marker image G1a is projected, a contrast between projection marker G10a and the other region is maximized, and the marker position can be easily detected in the captured image (step S23).

Again with reference to FIG. 11, projection controller 250 first causes projector 220 to project such marker image G1a for automatic correction as described above (step S21). Next, projection controller 250 acquires a captured image of marker image G1a on the imaging coordinate (Xi, Yi) at the time of image capturing by camera 210 (step S22), and calculates a deviation amount of the marker position on the imaging coordinate (Xi, Yi) (step S23).

Next, on the basis of the calculated deviation amount, projection controller 250 sets initial correction information to position correction unit 253 and position correction unit 251 (step S24). In step S24, projection controller 250 calculates correction information for the imaging coordinate (Xi, Yi) such that the deviation amount of captured marker Im10 on the imaging coordinate (Xi, Yi) corrected by position correction unit 253 and position correction unit 251 is equal to or less than an upper limit value representing the tolerance. The correction information is defined by, for example, various parameters representing coordinate transformation with respect to the imaging coordinate (Xi, Yi), and includes, for example, parameters of translation, rotation, and enlargement and reduction.

After initial setting of position correction unit 253 and position correction unit 251 (step S24), projection controller 250 ends step S10 in FIG. 7 and proceeds to step S11.

By the above automatic correction process, the imaging coordinate (Xi, Yi) is automatically corrected with projection marker G10a projected from the projector 220 used as reference, and position adjustment can therefore be easily performed in surgical operation support system 100.

The above automatic correction process may be performed while the superimposed image is being displayed on monitor 160 in the same manner as in step S14 in FIG. 7. At this time, projection controller 250 may cause monitor 160 to display the deviation amount in the same manner as in FIG. 10. Further, in steps S23 and S24, projection controller 250 may set position correction unit 253 and position correction unit 251 while gradually changing various parameters of the correction information.

### 2-2-3. Marker image and position adjustment method

During the operation in the position adjustment mode illustrated as an example in FIG. 7, after the automatic correction process (step S10), marker image G1 for position adjustment by user operation is projected instead of marker image G1 for automatic correction (step S11).

In marker image G1 in step S11, as illustrated as an example in FIG. 8, projection marker G10 further includes guide lines G11 for rotation adjustment and guide lines G12 for scaling adjustment in addition to marker points P0 to P4 similar to those in FIG. 12. Each of guide lines G11 and G12 is set to the same color as marker points P0 to P4, for example.

Guide lines G11 for rotation adjustment are provided radially from center marker point P0. Guide lines G12 for scaling adjustment are provided in a rectangular shape having four marker points P1 to P4 at four corners of the rectangular shape. Guide lines G11 and G12 are each provided at predetermined intervals from marker points P0 to P4. For example, in consideration of a tolerance in the automatic correction process (step S10), the predetermined intervals are set to a range larger than an upper limit value of the deviation amount expected between the reference position of projection marker G10 and the marker position of captured marker Im10.

Further, when superimposed image Im2 using projection marker G10 is displayed in step S14, projection controller 250 sets a color of projection marker G10 to, for example, a color different from that at the time of projection of marker G10 (step S11) (see FIG. 10). For example, projection marker G10 is set to black in step S11 and is set to light blue in step S14. In this case, captured marker Im10 appears black in superimposed image Im2, and projection marker G10 and captured marker Im10 can therefore be easily distinguished. Such a display method for distinguishing markers G10 and Im10 in superimposed image Im2 is not limited to the above method, and for example, change in line type, blinking display, or the like may be adopted.

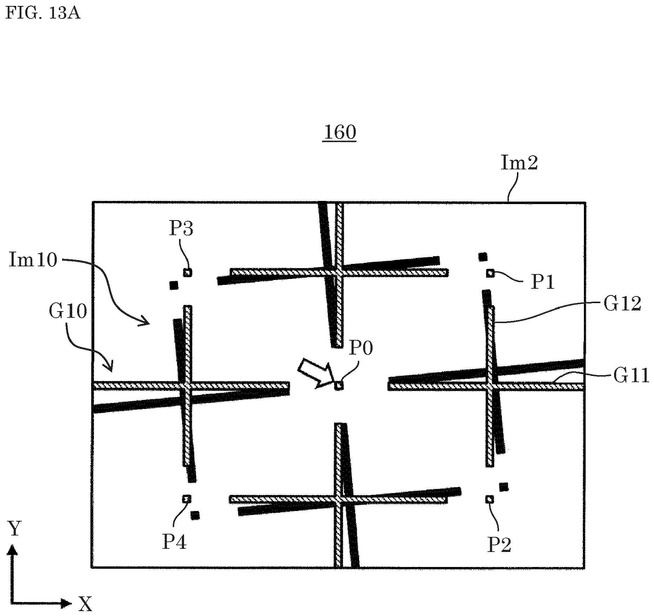

An example of a method of position adjustment by a user operation using projection marker G10 as described above will be described with reference to FIGS. 13A to 13D. FIGS. 13A to 13D illustrate display examples in the first to fourth position adjustment operations. Hereinafter, a description will be given on an example of a position adjustment method in which the process of steps S12 to S15 are repeated by a user operation.

FIG. 13A illustrates an example of a state in which the first position adjustment operation is performed after superimposed display illustrated in FIG. 10 is performed. In this example, as an operation of the first position adjustment, positioning in the X and Y directions is performed. At this time, positioning in the X and Y directions can be easily performed by shifting captured marker Im10 in the X direction and the Y direction taking center marker point P0 of projection marker G10 as a reference such that a position of a marker, in captured marker Im10, corresponding to center marker point P0 coincides with center marker point P0 of projection marker G10.

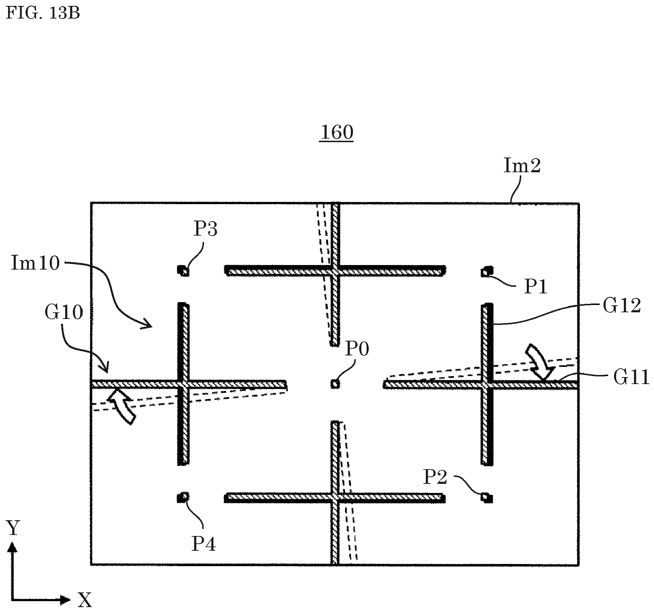

Next, in the second position adjustment operation, captured marker Im10 is rotated about, for example, the matched marker point P0 as illustrated in FIG. 13B. Such adjustment can be easily performed by paying attention to guide lines G11 for rotation adjustment radially disposed from marker point P0.

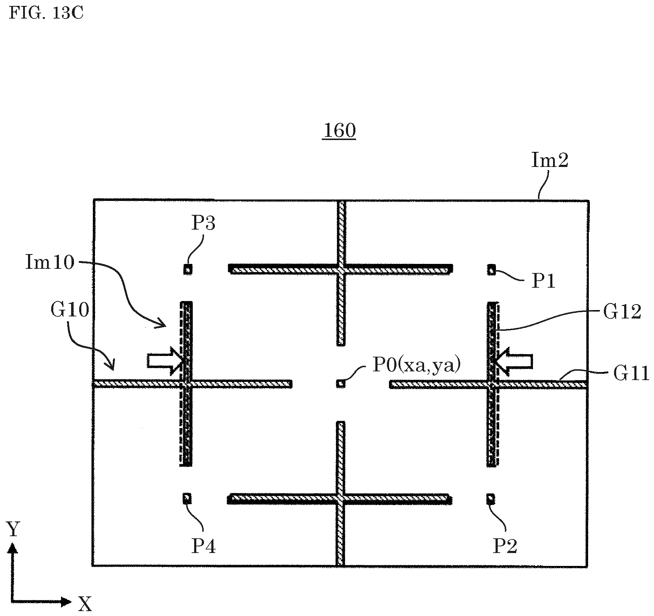

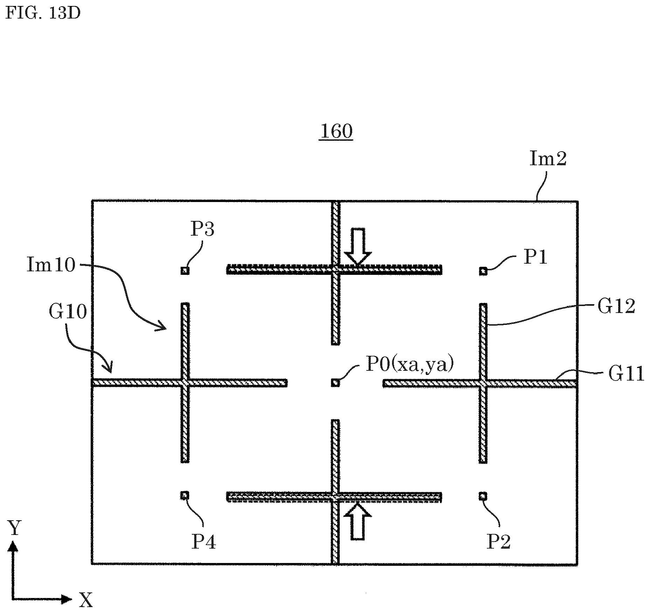

Further, for example, as illustrated in FIGS. 13C and 13D, the third and fourth position adjustments are performed in which captured marker Im10 is enlarged or reduced while keeping marker point P0 at a fixed position. Such position adjustments can be easily performed by paying attention to guide lines G12 for scaling adjustment disposed to surround marker point P0. The adjustment of the enlargement and reduction may be performed separately in the X direction and the Y direction. Alternatively, enlargement or reduction may be performed simultaneously in both the X and Y directions such that the guide lines in one direction coincide with each other, and then, fine adjustment may be performed in the other direction.

The position adjustment method as described above is limited to being performed by a user operation.

At the time of the position adjustment as described above, the deviation amounts (ΔX, ΔY) may be displayed in the same manner as in FIG. 10. In this case, projection controller 250 sequentially calculates the deviation amounts (step S13) to update the displayed deviation amounts. At this time, projection controller 250 may change display form depending on magnitudes of the deviation amounts. For example, when a deviation amount is equal to or more than a predetermined threshold value, the deviation amount may be displayed in red, and when a deviation amount is less than the threshold value, the deviation amount may be displayed in green.

### 2-3. Interpolation method for position adjustment

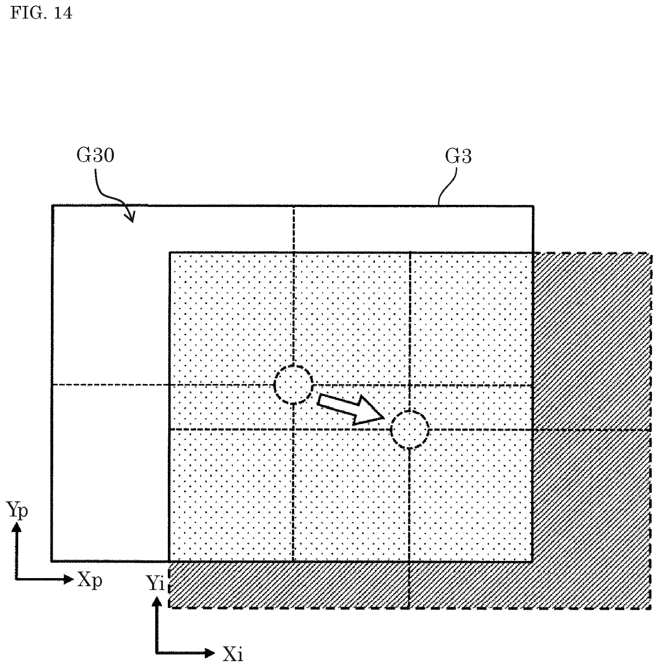

FIG. 14 is a diagram illustrating an interpolation method of projection image G3 in the normal mode of surgical operation support system 100.

In the normal mode of the present exemplary embodiment, position correction unit 251 of projection controller 250 performs coordinate transformation from the imaging coordinate (Xi, Yi) to the projection coordinate (Xp, Yp) according to the setting of the position adjustment mode as described above, so that position correction is performed such that the positions of the portions in captured image Im1 are wholly shifted on projection image G3. At this time, as illustrated in FIG. 14, in the whole area of the projection coordinate (Xp, Yp), there can be generated blank area G30 that does not correspond to captured image Im1.

Therefore, image generator 252 according to the present exemplary embodiment generates projection image G3 in which blank area G30 as described above is interpolated to be set white. As a result, it is possible to use projection light emitted in accordance with blank area G30 in projection image G3 at the time of projection from projector 220 as illumination for operative field 135 and the like.

In addition, image generator 252 sets also a region to white that is a region in projection image G3 and is other than the portion identified, by binarization or the like, to correspond to affected part 130. This makes it easy to secure illumination utilizing the projection light.

In the example described above, when blank area G30 is generated in accordance with the setting of the position adjustment mode, blank area G30 is interpolated with white. Such interpolation can also be applied to a case where blank area G30 is generated when the position of the image data on the projection coordinate (Xp, Yp) is moved not in accordance with the setting of the position adjustment mode but for various purposes.

Further, when such an image as described above is generated, it is possible to use, instead of white, such a middle tone usable as illumination light having higher luminance than an area corresponding to the captured image.

### 3. Conclusion

As described above, surgical operation support system 100 according to the present exemplary embodiment includes camera 210 that is an example of an imaging unit, projector 220 that is an example of a projection unit, and projection controller 250 that is an example of a controller. Camera 210 captures an image of a subject such as affected part 130 to generate a first captured image (step S2). Projector 220 projects projection image G320 corresponding to the first captured image onto the subject (step S4). Projection controller 250 has a normal mode and a position adjustment mode. The normal mode is an example of a first operation mode for generating a projection image in accordance with the first captured image. The position adjustment mode is an example of a second operation mode for adjusting a positional relationship in which a position in the first captured image and a position in the projection image are brought into correspondence with each other. In the position adjustment mode, projection controller 250 causes projector 220 to project marker image G1 including projection marker G10 as an example of a marker indicating a reference in the positional relationship (step S11). Projection controller 250 acquires from camera 210 captured image Im1 (second captured image) obtained by capturing an image of projected marker image G1 (step S12). Projection controller 250 adjusts the above positional relationship based on projection marker G10 in marker image G1 and the marker in captured image Im1, in other words, captured marker Im10 (steps S15 and S16).

With a projection system such as surgical operation support system 100 described above, marker image G1 projected from projector 220 is used as the reference, and the positional relationship between projection image G320 and the captured image in the normal mode can be easily adjusted.

In the present exemplary embodiment, in the normal mode, projection controller 250 operates as position correction unit 251 and image generator 252 and refers to the positional relationship adjusted in the position adjustment mode to generate projection image G320 in such a manner that a position in the first captured image and a position in projection image G320 are brought into correspondence with each other (step S3).

In the present exemplary embodiment, surgical operation support system 100 further includes monitor 160 that is an example of a display unit that displays an image. In the position adjustment mode, projection controller 250 controls and causes monitor 160 to display projection marker G10 in marker image G1, in a superimposed manner, on captured image Im1 (step S14). As a result, user 140 can check a positional deviation of captured marker Im10 based on projection marker G10 on monitor 160, and can easily perform position adjustment.

In the present exemplary embodiment, surgical operation support system 100 further includes operation unit 170 that inputs a user operation. In the position adjustment mode, projection controller 250 adjusts the positional relationship according to a user operation that is input from operation unit 170 (steps S15 and S16). With this system 100, user 140 can easily perform such position adjustment as to obtain a desired positional relationship.

In the present exemplary embodiment, in the position adjustment mode, projection controller 250 calculates a deviation amount between the position of projection marker G10 in marker image G1 and the position of captured marker Im10 in captured image Im1 (step S13). This system 100 may display the calculated deviation amount on monitor 160.

In the present exemplary embodiment, camera 210 captures an invisible light image that is an example of the first captured image on the basis of infrared light that is an example of first light having a first wavelength band. Further, camera 210 captures captured image Im1 as a visible light image that is an example of the second captured image on the basis of visible light that is an example of second light having a second wavelength band different from the first wavelength band. Surgical operation support system 100 can achieve various supports using the first and second captured images by camera 210.

In the present exemplary embodiment, the subject is a living body such as a patient, and includes affected part 130 that emits fluorescent light in the first wavelength band. Surgical operation support system 100 further includes excitation light source 230 that is an example of a light source unit. Excitation light source 230 emits excitation light 300 that excites fluorescence emission. With such surgical operation support system 100, it is possible to cause projection image G320 to visualize affected part 130 emitting fluorescent light and therefore to support a surgical operation or the like.

In the present exemplary embodiment, projection marker G10 has luminance lower than luminance of a region other than projection marker G10 in marker image G1. For example, projection marker G10 is set to black. With such projection marker G10, it is easy to see where the projection marker is projected, and position adjustment can therefore be easily performed.

In the present exemplary embodiment, as illustrated in FIG. 14, for example, projection controller 250 sets, in projection image G3, higher luminance to a position such as blank area G30 that does not correspond to the captured image than to a position that corresponds to the captured image. For example, projection controller 250 sets the luminance of the blank area G30 to white. With such projection image G3, the projection light on blank area G30 can be used for illumination.

### (Other exemplary embodiments)

The first exemplary embodiment has been described above as an example of techniques disclosed in the present application. However, the techniques in the present disclosure are not limited to the above exemplary embodiment and can also be applied to an exemplary embodiment in which modification, replacement, addition, removal, or the like is performed appropriately. Further, a new exemplary embodiment can also be made by a combination of the components of the first exemplary embodiment. Therefore, other exemplary embodiments will be described below as examples.

In the example described in the first exemplary embodiment, the automatic correction process (step S10 in FIG. 7) is performed during the operation of the position adjustment mode, but the automatic correction process may be omitted. Also with the above arrangements, it is possible to make it easy to adjust the positional relationship between the captured image and the projection image by using projection markers G10 and G10a as references.

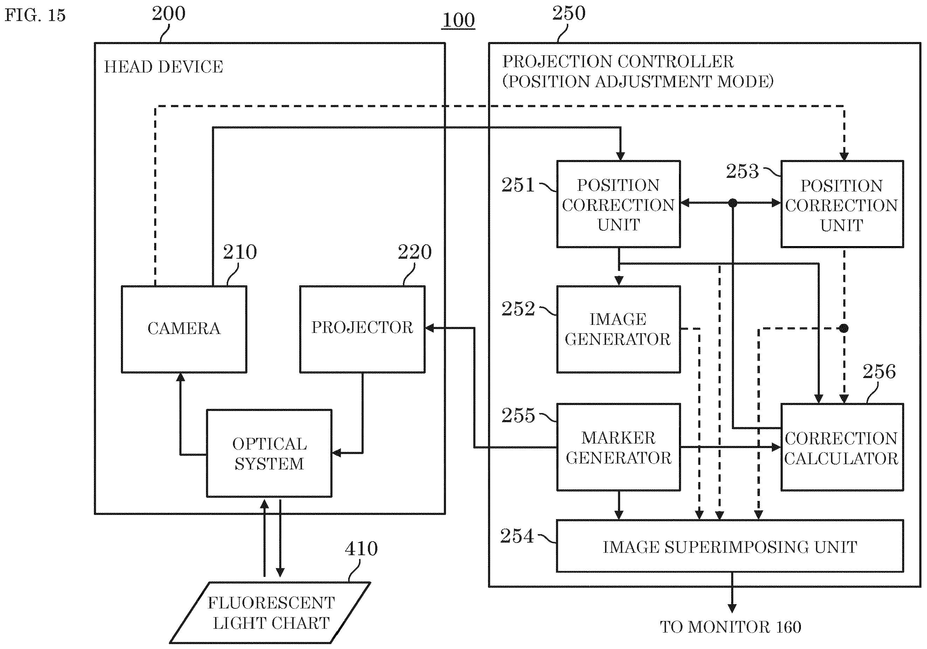

In the example described in the first exemplary embodiment, a visible light image is used for the position adjustment mode, but the position adjustment mode may be executed using an invisible light image instead of a visible light image. A first variation will be described with reference to FIG. 15.

FIG. 15 is a functional block diagram illustrating the first variation of the position adjustment mode in surgical operation support system 100. In the present variation, for example, instead of white chart 400 of FIG. 6, fluorescent light chart 410 is used as the subject. Fluorescent light chart 410 is made of a material that totally reflects a wavelength component of 830 nm that can be included in white projection light, for example. Further, in the present variation, in FIG. 6, calculation of the deviation amount and the like are performed with respect to the correction result of position correction unit 251 for an invisible light image instead of the correction result of position correction unit 253 for a visible light image.

It is possible to use both the position adjustment mode of the present variation as described above and the position adjustment mode of the first exemplary embodiment. With this arrangement, there can be set a difference in the correction information between position correction unit 251 for an invisible light image and position correction unit 253 for a visible light image. With such a difference in the correction information, it is possible to address the chromatic aberration of magnification between the visible light image and the invisible light image. The difference in the correction information may be set by the position adjustment mode of the present variation, for example, at the time of factory shipment.

As described above, in the position adjustment mode, projection controller 250 may acquire a third captured image in which the marker image projected from projector 220 is captured by camera 210 on the basis of the first light such as invisible light. On the basis of the acquired third captured image, projection controller 250 may set a difference between the following positional relationships: a positional relationship associating a position in an invisible light image (the first captured image) with a position in the projection image; and a positional relationship associating a position in a visible light image (the second captured image) with a position in the marker image.

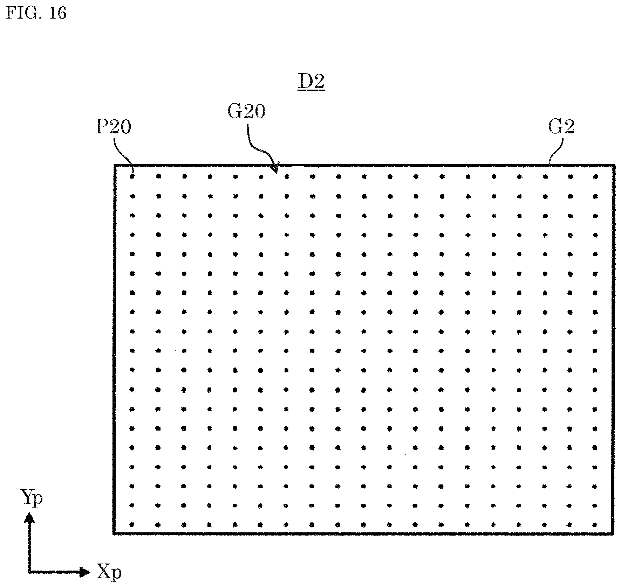

In the above exemplary embodiment, specific examples of marker images G1 and G1a have been described, but marker images G1 and G1a are not limited to the above examples, and various forms can be adopted. Such second variation as mentioned above will be described with reference to FIG. 16.

FIG. 16 is a diagram illustrating an example of image data D2 for projecting marker image G2 according to the second variation. Marker image G2 of the present variation includes a plurality of marker points P20 arranged in a grid shape as projection marker G20. For example, 20 × 20 marker points P20 are arranged at predetermined intervals over the entire projection coordinate (Xp, Yp). Note that the form of projection marker G20 is not particularly limited to the above, and for example, a number of marker points P20 other than the above may be set, or markers in various forms may be disposed instead of or in addition to marker points P20.

With projection marker G20 of the present variation, for example, in a case where distortion occurs in the projection image (or the captured image), a distortion amount can be measured by comparing, in the same manner as in the above-described exemplary embodiments, projection marker G20 with the corresponding captured marker. At this time, the distortion amount can be measured at each of the various places on the projection coordinate (Xp, Yp) where marker points P20 are disposed.

For example, it can be thought that there is a case where different position correction amounts need to be set between a central part and a peripheral part on the projection coordinate (Xp, Yp) due to an influence of distortion aberration of a lens in various optical systems. To address this case, by projecting projection marker G20 of the present variation and considering the distortion amount in a position adjustment mode similar to the position adjustment modes of the above exemplary embodiments, it is possible to perform nonlinear correction like for distortion aberration. In addition to uniform position correction (that is, translation, rotation, and scaling) for the entire projection coordinate (Xp, Yp), it is possible to perform local position correction for each grid.

In the above exemplary embodiments, infrared light has been described as an example of invisible light. However, the invisible light is not limited to infrared light, and may be ultraviolet light. In addition, the invisible light is not necessarily limited to light having a wavelength band in an invisible region, and may include, for example, weak fluorescent light in a red region that is emitted based on excitation light in a blue region. At this time, intended visible light for the projection image and a visible captured image may be green light or another color.

In the case described in the above exemplary embodiments, a time of the operation of the normal mode and a time of the operation of the position adjustment mode are different from each other. However, both modes are not necessarily performed at different times of operation and may be performed at the same time. For example, the imaging unit, the projection unit, and the controller of the present exemplary embodiment may be configured such that the first light used for capturing an image in the normal mode is in an infrared region and the second light used for capturing an image in the position adjustment mode is in an ultraviolet region. By differentiating the wavelength band of the light used for each operation mode as described above, it is possible to simultaneously perform the normal mode and the position adjustment mode. Further, by simultaneously performing as described above, position correction can be performed such that the position is adjusted in real time during a surgical operation.

In the above exemplary embodiment, an application example of the projection system in medical use has been described; however, the projection system in the present disclosure is not limited the above application example. In a case where it is necessary to perform work on an object whose state change cannot be visually checked, for example, a construction site, a mining site, a building site, or a factory for processing materials, the projection system according to the present disclosure can be applied.

Specifically, in a construction site, a mining site, a building site, a factory for processing materials, or the like, a fluorescent material may be applied to, kneaded in, or poured into an object whose state change cannot be visually confirmed, and the object may be captured as a subject for camera 210.

As described above, the exemplary embodiments have been described as examples of the techniques of the present disclosure. For that purpose, the accompanying drawings and the detailed description have been provided.

Therefore, in order to illustrate the above techniques as an example, the components described in the accompanying drawings and the detailed description can include not only components necessary to solve the problem but also components unnecessary to solve the problem. For this reason, it should not be immediately recognized that those unnecessary components are necessary just because those unnecessary components are described in the accompanying drawings and the detailed description.

Note that the exemplary embodiments described above are provided to describe, as an example, the techniques in the present disclosure.

### INDUSTRIAL APPLICABILITY

The projection system according to the present disclosure can be applied to work on a subject whose state change is difficult to be visually checked, for example, a medical application, a construction site, a mining site, a building site, or a material processing factory.

### REFERENCE MARKS IN THE DRAWINGS

100 surgical operation support system
130 affected part
135 operative field
150 display controller
160 monitor
170 operation unit
210 camera
220 projector
230 excitation light source
250 projection controller
251, 253 position correction unit
252 image generator
254 image superimposing unit
255 marker generator
256 correction calculator
300 excitation light
310 fluorescent light
315, 320 projection light
330 visible light
D1, D1a, D2 image data
G1, G1a, G2 marker image
G3, G320 projection image
G10, G10a, G20 projection marker
G30 blank area
Im1 captured image
Im2 superimposed image
Im10 captured marker
P0, P1, P2, P3, P4, P20 marker point

## Claims

1. A projection system (100) comprising:
an imaging unit (210) that captures a subject (120) to generate a first captured image;
a projection unit (220) projects a projection image (G3, G320) corresponding to the first captured image onto the subject (120);
a display unit (160) that displays an image,
an operation unit (170) that inputs a user operation, and
a controller (250) having a first operation mode and a second operation mode, the first operation mode being a mode to generate the projection image (G3, G320) corresponding to the first captured image and to cause the projection unit (220) to project the projection image (G3, G320), and the second operation mode being a mode to adjust a positional relationship that brings a position in the first captured image and a position in the projection image (G3, G320) into correspondence with each other,
**characterized in that**
in the second operation mode, the controller (250) causes the projection unit (220) to project a marker image (G1, G1a, G2) including a marker (P0, P1, P2, P3, P4, P20) indicating a reference in the positional relationship, acquires a second captured image (Im1) generated by the imaging unit (210) capturing an image of the projected marker image (G1, G1a, G2), and adjusts the positional relationship, based on the marker (P0, P1, P2, P3, P4, P20) in the marker image (G1, G1a, G2) and the marker in the second captured image (Im1)
in the first operation mode, referring to the positional relationship adjusted in the second operation mode, the controller (250) generates the projection image (G3, G320) where the position in the first captured image and the position in the projection image (G3, G320) are in correspondence with each other,
in the second operation mode, the controller (250) controls and causes the display unit (160) to display the marker (P0, P1, P2, P3, P4, P20) in the marker image (G1, G1a, G2), in a superimposed manner, on the second captured image (Im1), and
in the second operation mode, the controller (250) adjusts the positiona relationship according to the user operation that is input from the operation unit (170).

2. The projection system (100) according to claim 1, wherein in the second operation mode, the controller (250) calculates a deviation amount between a position of the marker (P0, P1, P2, P3, P4, P20) in the marker image (G1, G1a, G2) and a position of the marker (P0, P1, P2, P3, P4, P20) in the second captured image (Im2).

3. The projection system (100) according to any one of claims 1 and 2, wherein the imaging unit (210)
generates the first captured image by capturing an image of the subject (120), based on first light having a first wavelength band, and
generates the second captured image (Im1) by capturing an image of the marker image (G1, G1a, G2), based on second light having a second wavelength band different from the first wavelength band.

4. The projection system (100) according to claim 3, further comprising a light source unit (230) that emits excitation light,
wherein the subject (120) includes a region (130) that emits fluorescent light due to the excitation light.

5. The projection system (100) according to any one of claims 1 to 4, wherein the marker (P0, P1, P2, P3, P4, P20) has luminance lower than luminance of a region, in the marker image (G1, G1a, G2), other than the marker (P0, P1, P2, P3, P4, P20).

6. The projection system (100) according to any one of claims 1 to 5, wherein the controller (250) sets, in the projection image (G3, G320), higher luminance to a position not corresponding to the first captured image than a position corresponding to the first captured image.

## Patentansprüche

1. Projektionssystem (100), mit:
einer Bildaufnahmeeinheit (210), die ein Subjekt (120) aufnimmt, um ein erstes aufgenommenes Bild zu erzeugen,
einer Projektionseinheit (220), die ein Projektionsbild (G3, G320) entsprechend dem ersten aufgenommenen Bild auf das Subjekt (120) projiziert,
einer Anzeigeeinheit (160), die ein Bild anzeigt,
einer Bedienungseinheit (170), in die eine Benutzerbedienung eingegeben wird, und
einer Steuereinheit (250), die einen ersten Betriebsmodus und einen zweiten Betriebsmodus aufweist, wobei der erste Betriebsmodus ein Modus zum Erzeugen des Projektionsbildes (G3, G320) entsprechend dem ersten aufgenommenen Bild und zum Veranlassen der Projektionseinheit (220) zum Projizieren des Projektionsbildes (G3, G320) ist und der zweite Betriebsmodus ein Modus zum Justieren einer Positionsbeziehung ist, die eine Position in dem ersten aufgenommenen Bild und eine Position in dem Projektionsbild (G3, G320) in Entsprechung miteinander bringt,
**gekennzeichnet dadurch, dass**
in dem zweiten Betriebsmodus die Steuereinheit (250) die Projektionseinheit (220) veranlasst, ein Markierungsbild (G1, G1a, G2) mit einer Markierung (P0, P1, P2, P3, P4, P20) zu projizieren, das eine Referenz in der Positionsbeziehung angibt, ein zweites aufgenommenes Bild (Im1) erhält, das durch die Bildaufnahmeeinheit (210) erzeugt wurde, die ein Bild des projizierten Markierungsbildes (G1, G1a, G2) aufnimmt, und die Positionsbeziehung auf Basis der Markierung (P0, P1, P2, P3, P4, P20) in dem Markierungsbild (G1, G1a, G2) und der Markierung in dem zweiten aufgenommenen Bild (Im1) justiert,
in dem ersten Betriebsmodus die Steuereinheit (250) unter Bezugnahme auf die in dem zweiten Betriebsmodus justierte Positionsbeziehung das Projektionsbild (G3, G320) erzeugt, wobei die Position in dem ersten aufgenommenen Bild und die Position in dem Projektionsbild (G3, G320) in Entsprechung miteinander sind,
in dem zweiten Betriebsmodus die Steuereinheit (250) die Anzeigeeinheit (160) steuert und dazu veranlasst, die Markierung (P0, P1, P2, P3, P4, P20) in dem Markierungsbild (G1, G1a, G2) in einer überlagerten Weise auf dem zweiten aufgenommenen Bild (Im1) anzuzeigen, und
in dem zweiten Betriebsmodus die Steuereinheit (250) die Positionsbeziehung entsprechend der in die Bedienungseinheit (170) eingegebenen Benutzerbedienung justiert.

2. Projektionssystem (100) nach Anspruch 1, wobei in dem zweiten Betriebsmodus die Steuereinheit (250) eine Abweichung zwischen einer Position der Markierung (P0, P1, P2, P3, P4, P20) in dem Markierungsbild (G1, G1a, G2) und einer Position der Markierung (P0, P1, P2, P3, P4, P20) in dem zweiten aufgenommenen Bild (Im2) berechnet.

3. Projektionssystem (100) nach einem der Ansprüche 1 und 2, wobei die Bildaufnahmeeinheit (210)
das erste aufgenommene Bild durch Aufnehmen eines Bildes des Subjekts (120) auf Basis von erstem Licht mit einem ersten Wellenlängenband erzeugt, und
das zweite aufgenommene Bild (Im1) durch Aufnehmen eines Bildes des Markierungsbildes (G1, G1a, G2) auf Basis von zweitem Licht mit einem zweiten Wellenlängenband unterschiedlich zum ersten Wellenlängenband erzeugt.

4. Projektionssystem (100) nach Anspruch 3, ferner mit einer Lichtquelleneinheit (230), die Anregungslicht emittiert,
wobei das Subjekt (120) einen Bereich (130) aufweist, der Fluoreszenzlicht in Folge des Anregungslichts emittiert.

5. Projektionssystem (100) nach einem der Ansprüche 1 bis 4, wobei die Markierung (P0, P1, P2, P3, P4, P20) eine Luminanz geringer als eine Luminanz eines Bereichs in dem Markierungsbild (G1, G1a, G2) außerhalb der Markierung (P0, P1, P2, P3, P4, P20) aufweist.

6. Projektionssystem (100) nach einem der Ansprüche 1 bis 5, wobei die Steuereinheit (250) in dem Projektionsbild (G3, G320) eine höhere Luminanz an einer Position einstellt, die nicht dem ersten aufgenommenen Bild entspricht, im Vergleich zu einer Position, die dem ersten aufgenommenen Bild entspricht.

## Revendications

1. Système de projection (100) comprenant:
une unité de capture d'image (210) qui capture un sujet (120) pour générer une première image capturée,
une unité de projection (220) qui projette une image de projection (G3, G320) correspondant à la première image capturée sur le sujet (120),
une unité d'affichage (160) qui affiche une image,
une unité de maniement (170) dans laquelle est entré un maniement d'utilisateur, et
une unité de commande (250) ayant un premier mode de fonctionnement et un deuxième mode de fonctionnement, le premier mode de fonctionnement étant un mode pour générer l'image de projection (G3, G320) correspondant à la première image capturée et pour amener l'unité de projection (220) à projeter l'image de projection (G3, G320), et le deuxième mode de fonctionnement étant un mode pour ajuster une relation de position qui met en correspondance l'une avec l'autre une position dans la première image capturée et une position dans l'image de projection (G3, G320),
**caractérisé par le fait que**
dans le deuxième mode de fonctionnement, l'unité de commande (250) amène l'unité de projection (220) à projeter une image de marquage (G1, G1a, G2) comprenant un marquage (P0, P1, P2, P3, P4, P20) indiquant une référence dans la relation de position, acquiert une deuxième image capturée (Im1) générée par l'unité de capture d'image (210) capturant une image de l'image de marquage projetée (G1, G1a, G2), et ajuste la relation de position, sur la base du marquage (P0, P1, P2, P3, P4, P20) dans l'image de marquage (G1, G1a, G2) et le marquage dans la deuxième image capturée (Im1),
dans le premier mode de fonctionnement, en faisant référence à la relation de position ajustée dans le deuxième mode de fonctionnement, l'unité de commande (250) génère l'image de projection (G3, G320) où la position dans la première image capturée et la position dans l'image de projection (G3, G320) sont en correspondance l'une avec l'autre,
dans le deuxième mode de fonctionnement, l'unité de commande (250) commande l'unité d'affichage (160) et amène celle-ci à afficher le marquage (P0, P1, P2, P3, P4, P20) dans l'image de marquage (G1, G1a, G2), d'une manière superposée, sur la deuxième image capturée (Im1), et
dans le deuxième mode de fonctionnement, l'unité de commande (250) ajuste la relation de position selon le maniement d'utilisateur qui est entré dans l'unité de maniement (170).

2. Système de projection (100) selon la revendication 1, dans lequel, dans le deuxième mode de fonctionnement, l'unité de commande (250) calcule un écart entre une position du marquage (P0, P1, P2, P3, P4, P20) dans l'image de marquage (G1, G1a, G2) et une position du marquage (P0, P1, P2, P3, P4, P20) dans la deuxième image capturée (Im2).

3. Système de projection (100) selon l'une quelconque des revendications 1 et 2, dans lequel l'unité de capture d'image (210)
génère la première image capturée en capturant une image du sujet (120), sur la base d'une première lumière ayant une première bande de longueur d'onde, et
génère la deuxième image capturée (Im1) en capturant une image de l'image de marquage (G1, G1a, G2), sur la base d'une deuxième lumière ayant une deuxième bande de longueur d'onde différente de la première bande de longueur d'onde.

4. Système de projection (100) selon la revendication 3, comprenant en outre une unité de source de lumière (230) qui émet de la lumière d'excitation,
dans lequel le sujet (120) comprend une région (130) qui, dû à la lumière d'excitation, émet de la lumière fluorescente.

5. Système de projection (100) selon l'une quelconque des revendications 1 à 4, dans lequel le marquage (P0, P1, P2, P3, P4, P20) présente une luminance qui est inférieure à une luminance d'une région, dans l'image de marquage (G1, G1a, G2), autre que le marquage (P0, P1, P2, P3, P4, P20).

6. Système de projection (100) selon l'une quelconque des revendications 1 à 5, dans lequel l'unité de commande (250) règle, dans l'image de projection (G3, G320), une luminance plus élevée à une position qui ne correspond pas à la première image capturée qu'à une position qui correspond à la première image capturée.
